# FASCICULE DE BREVET EUROPEEN

(11) **EP 0 769 323 B1**
(45) Date de publication et mention de la délivrance du brevet: **30.01.2002**
(21) Numéro de dépôt: 96402202.4
(22) Date de dépôt: 15.10.1996
(51) Int. Cl.: B01J 31/14, B01J 23/36, C07C 6/04

(54) **Catalyseur supporté contenant du rhénium et de l'aluminium, procédé de préparation et application à la métathèse des oléfines**
Rhenium und Aluminium enthaltende Katalysatoren, ihre Herstellung und Verwendung für die Metathese von Olefinen
Rhenium and aluminium supported catalysts, their preparation and use for the metathesis of olefins

(30) Priorité: 20.10.1995 FR 9512335
(43) Date de publication de la demande: 23.04.1997
(73) Titulaire: INSTITUT FRANCAIS DU PETROLE, 92506 Rueil Malmaison Cédex (FR)
(72) Inventeur: Commereuc, Dominique, 92190 Meudon (FR)

(56) Documents cités:
- EP-A- 0 313 283
- EP-A- 0 639 549
- US-A- 4 454 368
- US-A- 4 943 397

## Description

La présente invention concerne un catalyseur à base de rhénium, un procédé de préparation du catalyseur et son application à la métathèse des oléfines.

La métathèse des oléfines, ou réaction de redistribution des groupements alkylidènes entre eux, présente un grand intérêt pratique, par exemple pour le rééquilibrage entre elles des oléfines légères issues du craquage à la vapeur, telles que l'éthylène, le propylène et les butènes.

Différents types de catalyseurs sont susceptibles d'être mis en oeuvre dans la réaction de métathèse, soit homogènes, quand leurs éléments constitutifs sont tous solubles dans le milieu de la réaction, soit hétérogènes, quand au moins un des éléments est insoluble dans le dit milieu. Ces derniers sont particulièrement intéressants quand le métal actif est onéreux et qu'il est nécessaire d'envisager sa réutilisation sans pertes. C'est le cas par exemple des catalyseurs à base de rhénium.

Les catalyseurs supportés à base de rhénium actifs pour la réaction de métathèse des oléfines sont peu nombreux. Le plus ancien et le plus couramment employé est l'heptoxyde de rhénium déposé sur des oxydes minéraux, le plus souvent sur de l'alumine (brevet GB-1.054.864, British Petroleum Co. Ltd, 1964), mais aussi sur des supports comportant à la fois de l'alumine et un autre co-oxyde (R. Nakamura, Rec. Trav. Chim. Pays-Bas, vol. 96, 1977, p. M31). L'utilisation de cocatalyseurs organométalliques, tels que les tétraalkyl-étains, associés au catalyseur oxyde de rhénium sur alumine, permet, entre autres avantages, la métathèse des oléfines fonctionnelles (J.C. Mol, C. Boelhouwer *et al*., J. Chem. Soc. Chem. Comm., 1977, p. 198). W. Herrmann a revendiqué la mise en oeuvre de complexes tels que le méthyltrioxorhénium et le pentaméthylcyclopentadiényl-trioxorhénium déposés sur des oxydes minéraux variés, dont surtout l'alumine et les silice-alumines (Hoechst, brevets EP-373.488-A, EP-522.067-A).

L'invention décrit un procédé de préparation permettant d'obtenir des catalyseurs modifiés à base de rhénium plus actifs que ceux de l'art antérieur et l'utilisation de ces'catalyseurs pour la métathèse des oléfines. Il a en effet été trouvé que l'addition à un catalyseur à base de rhénium supporté sur un support poreux minéral et calciné à une température de 200 à 1000 °C, d'au moins un composé particulier d'aluminium améliore de façon inattendue l'activité de ces catalyseurs, permettant d'utiliser, pour une activité identique, des proportions plus faibles de rhénium, ce qui est important au vu du coût de ce métal.

Le composé particulier d'aluminium a pour formule générale (RO)_{q}AlR'ᵣ, dans laquelle R est un reste hydrocarbyle contenant de 1 à 40 atomes de carbone, R' est un reste alkyle contenant de 1 à 20 atomes de carbone, q et r sont égaux à 1 ou 2 de telle façon que la somme q + r soit égale à 3.

Le catalyseur à base de rhénium, objet de l'invention, comprend au moins trois composants : au moins un support poreux minéral, 0,01 à 20 % en poids de rhénium (exprimé en métal) sous forme oxyde, et 0,01 à 10 % en poids d'aluminium introduit sous forme du composé d'aluminium de formule générale (RO)_{q}AlR'ᵣ.

L'invention concerne également des procédés de préparation du catalyseur. Plus précisément, dans un procédé de préparation objet de l'invention, on prépare un précurseur de catalyseur calciné contenant au moins un support poreux minéral et le rhénium, puis on met ledit précurseur au contact d'un composé (RO)_{q}AlR'ᵣ.

Le précurseur de catalyseur calciné est obtenu par introduction sur le(s) support(s) d'au moins un précurseur de rhénium, puis calcination.

Le support poreux minéral est choisi dans le groupe formé par des oxydes réfractaires et/ou des alumino-silicates qui peuvent avoir un caractère acide, neutre ou basique. On peut citer à titre d'exemple, sans que la liste soit limitative : l'alumine, la silice, les silice-alumines, les zéolithes, l'oxyde de titane, la zircone, l'oxyde de niobium, l'oxyde de chrome, la magnésie, l'oxyde d'étain.

On utilise avantageusement un support minéral à caractère acide ou neutre, plus particulièrement une alumine, une silice ou une silice-alumine, ayant une surface spécifique de 10 à 400 m²/g. De préférence, le support poreux est choisi dans le groupe formé par de l'alumine ou par un composé contenant au moins 75 % en poids d'alumine, qui doit avantageusement présenter une surface appréciable, par exemple au moins 10 m²/g, et de préférence au moins 50 m²/g, et un volume de pores suffisant, par exemple au moins 0,1 ml/g, et de préférence 0,3-1 ml/g. On peut utiliser par exemple une alumine du même type que celles des catalyseurs de reformage catalytique.

Le précurseur du composé de rhénium utilisé est choisi de préférence dans le groupe formé par l'heptoxyde de rhénium, le perrhénate d'ammonium et l'acide perrhénique. Le composé du rhénium peut être introduit sur le support par exemple par sublimation en phase vapeur ou par imprégnation en solution. On préfère généralement utiliser la méthode d'imprégnation à sec, où le composé du rhénium est mis en solution dans l'eau ou dans un solvant organique, par exemple un hydrocarbure, un alcool ou un éther. On règle la quantité de rhénium sur le support par le choix de la concentration de la solution d'imprégnation, sa quantité étant telle que le volume de cette solution soit égal ou légèrement inférieur au volume poreux du solide à imprégner. Lorsque la quantité de rhénium que l'on désire imprégner est supérieure à celle que permet d'introduire une solution à sa limite de saturation, on doit effectuer l'opération en plusieurs fois, avec des séchages intermédiaires pour éliminer le solvant d'imprégnation, à une température de par exemple 90 à 250 °C, de préférence 100 à 180 °C. Ceci permet d'introduire de 0,01 à 20 %, de préférence de 0,1 à 15 %, et encore plus avantageusement de 0,5 à 8 % en poids de rhénium (exprimé en rhénium métal).

Après l'introduction du précurseur de rhénium sur le support, on effectue un séchage à une température de par exemple 90 à 250 °C, de préférence 100 à 180 °C, puis une calcination à une température de 200 à 1000° C, par exemple 250 à 1000 °C, et de préférence 300 à 600 °C, pendant une durée de 10 minutes à 10 heures, et de préférence de 30 minutes à 5 heures. Après calcination, le solide est refroidi sous atmosphère sèche et inerte, par exemple sous azote ou sous argon.

Tout support existant chargé en oxyde de rhénium convient, et tout mode de préparation est acceptable. Aussi les catalyseurs à base de rhénium actuellement commercialisés peuvent convenir.

Le composé d'aluminium répond à la formule générale (RO)qAlR'r, dans laquelle R est un reste hydrocarbyle contenant de 1 à 40 atomes de carbone, par exemple alkyle, cycloalkyle, alkényle, aryle, aryle ou cycloalkyle substitués, de préférence un reste hydrocarbyle de 2 à 30 atomes de carbone, ce reste pouvant être substitué par au moins un groupe alkoxy ou au moins un halogène. A titre d'exemple, et sans que la liste soit limitative, R peut être un reste éthyle, n-propyle, isopropyle, n-butyle, t-butyle, cyclohexyle, benzyle, diphénylméthyle, phényle, méthyl-2-phényle, méthyl-4-phényle, méthoxy-2-phényle, méthoxy-4-phényle, diméthyl-2,6-phényle, diisopropyl-2,6-phényle, t-butyl-2-phényle, t-butyl-2-méthyl-4-phényle, di-t-butyl-2,6-phényle, di-t-butyl-2,6-méthyl-4-phényle, tri-t-butyl-2,4,6-phényle, phényl-2-phényle, diphényl-2,6-phényle, fluoro-2-phényle, fluoro-4-phényle, pentafluorophényle. R' est un reste alkyle contenant de 1 à 20 atomes de carbone, de préférence de 1 à 6 atomes de carbone, par exemple méthyle, éthyle, isobutyle, q et r sont égaux à 1 ou 2 de telle façon que la somme q + r soit égale à 3.

La préparation du composé (RO)_{q}AlR'ᵣ est connue dans la littérature. Tout procédé de préparation de ce composé convient. On peut par exemple faire réagir un alcool ou un phénol ROH avec un trialkylaluminium AlR'₃ dans un solvant organique, par exemple un hydrocarbure ou un éther.

Le composé de l'aluminium peut être introduit sur le support par toutes les méthodes connues de l'homme de l'art, mais il est impératif d'opérer à l'abri de l'air et de l'humidité. On peut imprégner le support par un excès d'une solution contenant le composé de l'aluminium (RO)_{q}AlR'ᵣ. Après un temps de contact qui peut aller de quelques minutes à quelques jours, on essore le solide et on le lave au solvant pour éliminer la portion du composé qui ne s'est pas fixée. On peut aussi, dans un mode opératoire qui est préféré, utiliser la méthode d'imprégnation à sec. On ajuste alors la concentration en aluminium de la solution en fonction de la quantité d'aluminium que l'on souhaite déposer sur le solide, de façon que le volume de cette solution soit égal ou légèrement inférieur au volume poreux du solide à imprégner. Le solvant mis en oeuvre dans cette imprégnation est de préférence un solvant organique, par exemple un hydrocarbure ou un éther. Ceci permet d'introduire de 0,01 à 10 %, de préférence de 0,05 à 5 %, et encore plus avantageusement de 0,1 à 5 % en poids d'aluminium (exprimé en aluminium métal).

Après introduction du composé de l'aluminium, la préparation du catalyseur peut être terminée par un séchage, sous vide ou sous un courant de gaz de préférence inerte, à une température de 0 à 1000° C, avantageusement inférieure à 200° C de façon à éviter la décomposition du composé d'aluminium, voire de 0 à 180° C, de préférence à une température voisine de l'ambiante, de 0 à 50° C. Aucune opération d'activation, chimique ou thermique, n'est nécessaire pour déclencher l'activité de ces catalyseurs, et la calcination est déconseillée. Il suffit de les mettre en contact avec une oléfine pour que la réaction de métathèse démarre.

Au lieu de préparer le composé (RO)_{q}AlR'ᵣ et de le mettre au contact du catalyseur supporté au rhénium, ainsi que précédemment décrit, on peut mettre ledit catalyseur supporté au rhénium directement en contact des réactifs précurseurs du composé (RO)_{q}AlR'ᵣ, qui sont par exemple ROH et AlR'₃ avec R, R' précédemment définis. De la même façon que précédemment, la préparation peut se terminer par un séchage.

L'addition du composé (RO)_{q}AlR'ᵣ ou de ses précurseurs sur le catalyseur au rhénium supporté peut ainsi avantageusement avoir lieu in situ dans le réacteur de réaction, avant la réaction, ou bien ex situ et le catalyseur modifié est directement chargé dans le réacteur pour la réaction. On peut dans le premier cas (in situ) également introduire le composé ou ses précurseurs avec la charge à traiter.

L'invention a aussi pour objet un procédé de métathèse des oléfines en présence du catalyseur défini ci-dessus, à une température de -20 à +200 °C, de préférence de 0 à +100 °C, dans des conditions de pression variables selon que l'on souhaite conduire la réaction en phase gazeuse ou en phase liquide.

Dans une opération en phase liquide, la pression doit être suffisante pour que les réactifs et le solvant éventuel soient maintenus au moins en majorité (plus de 50 %) en phase liquide (ou en phase condensée). Le catalyseur peut alors être mis en oeuvre soit dans l'oléfine (ou les oléfines) pure, soit en présence d'un solvant constitué par un hydrocarbure aliphatique, cycloaliphatique ou aromatique, un hydrocarbure halogéné ou un dérivé nitré. On utilise de préférence un hydrocarbure ou un hydrocarbure halogéné.

Les oléfines susceptibles de réagir en métathèse sont des monooléfines ayant de 2 à 30 atomes de carbone, par exemple l'éthylène, le propylène, les butènes, les pentènes, des cyclooléfines ayant de 3 à 20 atomes de carbone, par exemple le cyclopentène, le cyclooctène, le norbornène, des polyoléfines ayant de 4 à 30 atomes de carbone, par exemple l'hexadiène-1,4, l'octadiène-1,7, des cyclopolyoléfines ayant de 5 à 30 atomes de carbone, par exemple le cyclooctadiène-1,5, le norbornadiène, le dicyclopentadiène.

D'autres oléfines susceptibles de réagir en métathèse sont les monooléfines ou les polyoléfines, linéaires ou cycliques, portant des groupes fonctionnels comme par exemple des halogènes ou des groupes ester tels que l'oléate de méthyle. Le procédé peut également mettre en oeuvre, en cométathèse, un mélange des oléfines précédentes.

Les exemples suivants illustrent l'invention sans en limiter la portée.

### EXEMPLE 1

### Préparation du catalyseur :

Dans une première étape, on calcine à 300 °C sous air une alumine gamma cubique ayant une surface spécifique de 184 m²/g et un volume poreux de 0,67 ml/g. Après refroidissement à température ambiante, on prélève 10 g d'alumine calcinée. On prépare une solution pour l'imprégnation du rhénium en diluant 0,24 ml d'une solution aqueuse concentrée d'acide perrhénique contenant 54 % en poids de rhénium (masse spécifique : 2,4 g/ml) dans 5 ml d'eau. Cette solution est imprégnée sur les 10 g d'alumine prélevés. Après 30 minutes de contact à température ambiante, on sèche le solide obtenu dans une étuve à 120 °C pendant une nuit. On le calcine ensuite sous un courant d'air (environ 20 l/h) séché par passage à travers un lit de tamis moléculaire, à une température de 550 °C pendant 2 heures. Pendant la période ultérieure de refroidissement, on substitue au courant d'air un courant d'azote sec. Le solide obtenu est conservé et manipulé en atmosphère d'azote sec. Sa teneur en rhénium métal est de 3 % en poids.

Dans un ballon de 250 ml placé sous atmosphère d'argon et muni d'un barreau magnétique, on introduit une solution de 0,493 g de triisobutylaluminium dans 20 ml de pentane, puis on injecte goutte-à-goutte, sous agitation et à température ambiante, une solution de 1,095 g de di-t-butyl-2,6-méthyl-4-phénol dans 30 ml de pentane. Après environ 30 heures de réaction, le pentane est évaporé sous vide et l'analyse du solide blanc restant indique qu'il est constitué essentiellement par le bis-(di-t-butyl-2,6-méthyl-4-phénoxy)-isobutylaluminium. Ce composé est remis en solution dans 5 ml d'heptane.

La solution dans l'heptane du bis-(di-t-butyl-2,6-méthyl-4-phénoxy)-isobutylaluminium est alors imprégnée sur le solide contenant le rhénium obtenu dans la première étape. Après environ 30 minutes de contact, l'heptane absorbé sur le solide est éliminé par évaporation sous vide à température ambiante. On obtient ainsi un catalyseur de métathèse contenant 3 % en poids de rhénium et 0,67 % en poids d'aluminium (en sus de l'aluminium inclus dans l'alumine), qui est conservé en atmosphère sèche et inerte avant utilisation.

### Utilisation en métathèse :

Dans un réacteur constitué par un tube en acier inoxydable muni d'une double enveloppe avec circulation d'eau permettant la régulation de la température, on charge à l'abri de l'air et de l'humidité le catalyseur préparé ci-dessus. Du propylène liquide est injecté au moyen d'une pompe par le bas du réacteur, avec un débit de 49,6 g/h. La température est réglée à 35 °C et la pression est maintenue à 3,5 MPa au moyen d'un régulateur placé en aval du réacteur. Dans ces conditions, la conversion du propylène à la sortie du réacteur est de 30 %, en un mélange équimolaire d'éthylène et de butènes-2.

### EXEMPLE 2 (comparatif)

### Préparation du catalyseur :

Un nouveau lot de catalyseur est préparé comme dans l'exemple 1, à cette différence près que l'on omet l'étape de préparation et d'imprégnation par le bis-(di-t-butyl-2,6-méthyl-4-phénoxy)-isobutylaluminium. L'étape d'imprégnation du rhénium ainsi que la phase de séchage et de calcination sont identiques à celles décrites dans l'exemple 1. On obtient ainsi 10 g de catalyseur qui est conservé en atmosphère sèche et inerte avant utilisation. Sa teneur en rhénium métal est de 3 % en poids.

### Utilisation en métathèse :

Dans le même appareillage que celui décrit dans l'exemple 1, on charge les 10 g de catalyseur préparés ci-dessus. Du propylène liquide est injecté au moyen d'une pompe par le bas du réacteur, avec un débit de 49,6 g/h. La température est réglée à 35 °C et la pression est maintenue à 3,5 MPa au moyen d'un régulateur placé en aval du réacteur. Dans ces conditions, la conversion du propylène à la sortie du réacteur est de 7,4 %, en un mélange équimolaire d'éthylène et de butènes-2.

Cet exemple comparatif montre le progrès apporté par la méthode de préparation selon l'invention en ce qui concerne l'activité du catalyseur.

## Revendications

1. Catalyseur comprenant au moins un support poreux minéral, 0,01 à 20 % en poids de rhénium sous forme oxyde, et 0,01 à 10 % en poids d'aluminium introduit sous forme d'un composé d'aluminium de formule générale (RO)_{q}AlR'ᵣ, dans laquelle R est un reste hydrocarbyle contenant de 1 à 40 atomes de carbone, R' est un reste alkyle contenant de 1 à 20 atomes de carbone, q et r sont égaux à 1 ou 2 de telle façon que la somme q + r soit égale à 3.

2. Catalyseur selon la revendication 1, dans lequel le support poreux minéral est choisi dans le groupe formé par l'alumine, la silice, les silice-alumines, les zéolithes, l'oxyde de titane, la zircone, l'oxyde de niobium, l'oxyde de chrome, la magnésie, l'oxyde d'étain.

3. Catalyseur selon l'une des revendications 1 à 2, dans lequel le support poreux minéral est choisi dans le groupe formé par l'alumine et les composés contenant au moins 75 % en poids d'alumine, et présente une surface d'au moins 10 m²/g et un volume de pores d'au moins 0,1 ml/g.

4. Catalyseur selon l'une des revendications 1 à 3, dans lequel le composé d'aluminium répond à la formule générale (RO)_{q}AlR'ᵣ, dans laquelle R est un reste hydrocarbyle choisi dans le groupe formé par les restes alkyle, cycloalkyle, alkényle, aryle, aryle ou cycloalkyle substitués, ce reste pouvant être substitué par au moins un groupe alkoxy ou au moins un halogène.

5. Catalyseur selon l'une des revendications 1 à 4, dans lequel le composé (RO)_{q}AlR'ᵣ résulte de la réaction entre les composés ROH et AlR'₃.

6. Procédé de préparation d'un catalyseur modifié comprenant un support poreux minéral, de 0,01 à 20 % en poids de rhénium sous forme d'oxyde, et de 0,01 à 10 % en poids d'aluminium, dans lequel on prépare un précurseur de catalyseur calciné contenant au moins un support poreux minéral et le rhénium, puis on met ledit précurseur au contact d'un composé de formule générale (RO)_{q}AlR'ᵣ, dans laquelle R est un reste hydrocarbyle contenant de 1 à 40 atomes de carbone, R' est un reste alkyle contenant de 1 à 20 atomes de carbone, q et r sont égaux à 1 ou 2 de telle façon que q + r soit égal à 3, et le produit obtenu est séché.

7. Procédé de préparation d'un catalyseur modifié selon la revendication 6, dans lequel le composé (RO)qAlR'r résulte de la réaction entre un composé ROH et un composé AlR'₃.

8. Procédé de préparation d'un catalyseur modifié comprenant un support poreux minéral, de 0,01 à 20 % en poids de rhénium sous forme d'oxyde, et de 0,01 à 10 % en poids d'aluminium, dans lequel on prépare un précurseur calciné contenant le support poreux minéral et le rhénium, puis on met ledit précurseur au contact des réactifs précurseurs du composé de formule générale (RO)_{q}AlR'ᵣ, dans laquelle R est un reste hydrocarbyle contenant de 1 à 40 atomes de carbone, R' est un reste alkyle contenant de 1 à 20 atomes de carbone, q et r sont égaux à 1 ou 2 de telle façon que q + r soit égal à 3, et le produit obtenu est séché.

9. Procédé de préparation d'un catalyseur modifié selon la revendication 8, dans lequel les réactifs précurseurs du composé (RO)_{q}AlR'ᵣ sont les composés ROH et AlR'₃.

10. Procédé selon l'une des revendications 6 à 9, dans lequel le support poreux minéral est choisi dans le groupe formé par l'alumine, la silice, les silice-alumines, les zéolithes, l'oxyde de titane, la zircone, l'oxyde de niobium, l'oxyde de chrome, la magnésie, l'oxyde d'étain.

11. Procédé selon l'une des revendications 6 à 10, dans lequel le support poreux minéral est choisi dans le groupe formé par l'alumine et les composés contenant au moins 75 % en poids d'alumine, et présente une surface d'au moins 10 m²/g et un volume de pores d'au moins 0,1 ml/g.

12. Procédé selon l'une des revendications 6 à 11, dans lequel le précurseur de catalyseur est obtenu par contact du support avec au moins un composé de rhénium utilisé comme précurseur choisi dans le groupe formé par l'heptoxyde de rhénium, le perrhénate d'ammonium et l'acide perrhénique.

13. Procédé selon la revendication 12, **caractérisé en ce que**, après introduction du rhénium sur le support, on effectue une calcination à une température de 200 à 1000 °C, pendant une durée de 10 minutes à 10 heures.

14. Procédé selon l'une des revendications 6 à 13, **caractérisé en ce que** le composé d'aluminium répond à la formule générale (RO)_{q}AlR'ᵣ, dans laquelle R est un reste hydrocarbyle choisi dans le groupe formé par les restes alkyle, cycloalkyle, alkényle, aryle, aryle ou cycloalkyle substitués, ce reste pouvant être substitué par au moins un groupe alkoxy ou au moins un halogène.

15. Procédé selon l'une des revendications 6 à 14, **caractérisé en ce que** le produit obtenu après contact avec le composé de formule (RO)_{q}AlR'ᵣ ou avec ses précurseurs est séché à une température inférieure à 200 °C.

16. Procédé de métathèse des oléfines, **caractérisé en ce qu'**il opère à une température de -20 à +200 °C, et en présence d'un catalyseur selon l'une des revendications 1 à 5.

17. Procédé de métathèse des oléfines, **caractérisé en ce qu'**il opère à une température de -20 à +200 °C, et en présence d'un catalyseur obtenu selon l'une des revendications 6 à 14.

18. Procédé selon l'une des revendications 16 à 17, **caractérisé en ce qu'**il opère entre 0 et 100 °C.

19. Procédé selon l'une des revendications 16 à 18, **caractérisé en ce que** les oléfines sont choisies dans le groupe formé par les monooléfines ayant de 2 à 30 atomes de carbone, les cyclooléfines ayant de 3 à 20 atomes de carbone, les polyoléfines ayant de 4 à 30 atomes de carbone, les cyclopolyoléfines ayant de 5 à 30 atomes de carbone, les monooléfines ayant de 2 à 30 atomes de carbone portant des groupes fonctionnels choisis dans le groupe formé par les halogènes et les groupes ester, les polyoléfines ayant de 4 à 30 atomes de carbone portant des groupes fonctionnels choisis dans le groupe formé par les halogènes et les groupes ester.

20. Procédé selon l'une des revendications 16 à 19, **caractérisé en ce que** les oléfines sont choisies dans le groupe formé par l'éthylène, le propylène, les butènes, les pentènes, le cyclopentène, le cyclooctène, le norbornène, l'hexadiène-1,4, l'octadiène-1,7, le cyclooctadiène-1,5, le norbornadiène, le dicyclopentadiène, l'oléate de méthyle.

21. Procédé selon l'une des revendications 16 à 20, **caractérisé en ce qu'**il se déroule en phase gazeuse.

22. Procédé selon l'une des revendications 16 à 20, **caractérisé en ce que** les réactifs et le solvant éventuel sont au moins en partie en phase liquide.

## Claims

1. A catalyst comprising at least one inorganic porous support, from 0.01 to 20% by weight of rhenium in oxide form, and from 0.01 to 10% by weight of aluminium introduced in the form of an aluminium compound of the general formula (RO)_{q}AlR'ᵣ, wherein R is a hydrocarbyl residue containing from 1 to 40 carbon atoms, R' is an alkyl residue containing from 1 to 20 carbon atoms, and q and r are equal to 1 or 2 in such a way that the sum q+r is equal to 3.

2. A catalyst according to claim 1 wherein the inorganic porous support is selected from the group formed by alumina, silica, silica-aluminas, zeolites, titanium oxide, zirconia, niobium oxide, chromium oxide, magnesia and tin oxide.

3. A catalyst according to one of claims 1 and 2 wherein the inorganic porous support is selected from the group formed by alumina and compounds containing at least 75% by weight of alumina and has a surface area of at least 10 m²/g and a pore volume of at least 0.1 ml/g.

4. A catalyst according to one of claims 1 to 3 wherein the aluminium compound corresponds to the general formula (RO)_{q}AlR'ᵣ wherein R is a hydrocarbyl residue selected from the group formed by the residues: alkyl, cycloalkyl, alkenyl, aryl, aryl or cycloalkyl which are substituted, which residue can be substituted by at least one alkoxy group or at least one halogen.

5. A catalyst according to one of claims 1 to 4 wherein the compound (RO)_{q}AlR'ᵣ results from the reaction between the compounds ROH and AlR'₃.

6. A process for the preparation of a modified catalyst comprising an inorganic porous support, from 0.01 to 20% by weight of rhenium in oxide form, and from 0.01 to 10% by weight of aluminium, wherein there is prepared a roasted catalyst precursor containing at least one inorganic porous support and the rhenium, then said precursor is brought into contact with a compound of the general formula (RO)_{q}AlR'ᵣ wherein R is a hydrocarbyl residue containing from 1 to 40 carbon atoms, R' is an alkyl residue containing from 1 to 20 carbon atoms, and q and r are equal to 1 or 2 in such a way that q+r is equal to 3, and the product obtained is dried.

7. A process for the preparation of a modified catalyst according to claim 6 wherein the compound (RO)_{q}AlR'ᵣ results from the reaction between a compound ROH and a compound AlR'₃.

8. A process for the preparation of a modified catalyst comprising an inorganic porous support, from 0.01 to 20% by weight of rhenium in oxide form, and from 0.01 to 10% by weight of aluminium, wherein there is prepared a roasted precursor containing the inorganic porous support and the rhenium, then said precursor is brought into contact with the precursor reactants of the compound of the general formula (RO)_{q}AlR'ᵣ, wherein R is a hydrocarbyl residue containing from 1 to 40 carbon atoms, R' is an alkyl residue containing from 1 to 20 carbon atoms, and q and r are equal to 1 or 2 in such a way that q+r is equal to 3, and the product obtained is dried.

9. A process for the preparation of a modified catalyst according to claim 8 wherein the precursor reactants of the compound (RO)_{q}AlR'ᵣ are the compounds ROH and AlR'₃.

10. A process according to one of claims 6 to 9 wherein the inorganic porous support is selected from the group formed by alumina, silica, silica-aluminas, zeolites, titanium oxide, zirconia, niobium oxide, chromium oxide, magnesia and tin oxide.

11. A process according to one of claims 6 to 10 wherein the inorganic porous support is selected from the group formed by alumina and compounds containing at least 75% by weight of alumina, and has a surface area of at least 10 m²/g and a pore volume of at least 0.1 ml/g.

12. A process according to one of claims 6 to 11 wherein the catalyst precursor is obtained by contacting the support with at least one compound of rhenium used as a precursor selected from the group formed by rhenium heptoxide, ammonium perrhenate and perrhenic acid.

13. A process according to claim 12 **characterised in that**, after the rhenium has been introduced on to the support, roasting is effected at a temperature of from 200 to 1000°C for a period of from 10 minutes to 10 hours.

14. A process according to one of claims 6 to 13 **characterised in that** the aluminium compound corresponds to the general formula (RO)_{q}AlR'ᵣ wherein R is a hydrocarbyl residue selected from the group formed by the residues: alkyl, cycloalkyl, alkenyl, aryl, aryl or cycloalkyl which are substituted, which residue can be substituted by at least one alkoxy group or at least one halogen.

15. A process according of claims 6 to 14 **characterised in that** the product obtained after contacting the roasted catalyst precursor with the compound of formula (RO)_{q} AlR'ᵣ or with its precursors is dried at a temperature lower than 200°C.

16. A process for the metathesis of olefins **characterised in that** it operates at a temperature of from -20 to +200°C and in the presence of a catalyst according to one of claims 1 to 5.

17. A process for the metathesis of olefins **characterised in that** it operates at a temperature of from -20 to +200°C and in the presence of a catalyst obtained in accordance with one of claims 6 to 14.

18. A process according to one of claims 16 and 17 **characterised in that** it operates at between 0 and 100°C.

19. A process according to one of claims 16 to 18 **characterised in that** the olefins are selected from the group formed by monoolefins having from 2 to 30 carbon atoms, cycloolefins having from 3 to 20 carbon atoms, polyolefins having from 4 to 30 carbon atoms, cyclopolyolefins having from 5 to 30 carbon atoms, monoolefins having from 2 to 30 carbon atoms bearing functional groups selected from the group formed by halogens and ester groups, and polyolefins having from 4 to 30 carbon atoms bearing functional groups selected from the group formed by halogens and the ester groups.

20. A process according to one of claims 16 to 19 **characterised in that** the olefins are selected from the group formed by ethylene, propylene, butenes, pentenes, cyclopentene, cyclooctene, norbornene, hexa-1,4-diene, octa-1,7-diene, cycloocta-1,5-diene, norbornadiene, dicyclopentadiene and methyl oleate.

21. A process according to one of claims 16 to 20 **characterised in that** it takes place in a gaseous phase.

22. A process according to one of claims 16 to 20 **characterised in that** the reactants and the solvent if provided are at least in part in the liquid phase.

## Patentansprüche

1. Katalysator umfassend: wenigstens einen porösen mineralischen Träger, 0,01 bis 20 Gew.-% Rhenium in Form des Oxids und 0,01 bis 10 Gew.-% Aluminium, eingeführt in Form einer Aluminiumverbindung der allgemeinen Formel (RO)_{q}AlR'ᵣ, wo R ein Hydrocarbylrest, der 1 bis 40 Kohlenstoffatome enthält und R' ein Alkylrest, der 1 bis 20 Kohlenstoffatome enthält, ist und wobei q und r gleich 1 oder 2 derart sind, dass die Summe von q + r gleich 3 ist.

2. Katalysator nach Anspruch 1, bei dem der mineralische poröse Träger gewählt ist aus der Gruppe, die gebildet ist aus Aluminiumoxid, Siliziumoxid, den Siliziumoxiden-Aluminiumoxiden, den Zeolithen, dem Titanoxid, dem Zirkonium, dem Oxid des Niobs, dem Oxid des Chroms, dem Magnesiumoxid und dem Zinnoxid.

3. Katalysator nach einem der Ansprüche 1 bis 2, bei dem der mineralische poröse Träger gewählt ist aus der durch Aluminiumoxid und die Verbindungen gebildeten Gruppe, welche wenigstens 75 Gew.-% Aluminiumoxid enthalten und über eine Oberfläche von wenigstens 10 m²/g und ein Porenvolumen von wenigstens 0,1 ml/g, verfügt.

4. Katalysator nach einem der Ansprüche 1 bis 3, bei dem die Aluminiumverbindung der allgemeinen Formel (RO)_{q}AlR'ᵣ entspricht, in der R ein Hydrocarbylrest ist, der aus der durch die Alkyl-, Zykloalkyl-, Alkenyl-, Aryl-, Arlyl- oder Zykloalkylreste, die letzten beiden in Substitution, gebildeten Gruppe gewählt ist, wobei dieser Rest durch wenigstens eine Alkoxygruppe oder wenigstens ein Halogen substituiert werden kann.

5. Katalysator nach einem der Ansprüche 1 bis 4, bei dem die (RO)_{q}AlR'ᵣ aus der Reaktion zwischen den Verbindungen ROH und AlR'₃ resultiert.

6. Verfahren zur Herstellung eines modifizierten Katalysators, der einen porösen mineralischen Träger, zwischen 0,01 bis 20 Gew.-% Rhenium in Form des Oxids und zwischen 0,01 und 10 Gew.-% Aluminium umfasst, bei dem man einen kalzinierten Katalysatorvorläufer herstellt, der wenigstens einen mineralischen porösen Träger und Rhenium enthält und dann diesen Vorläufer in Kontakt mit einer Verbindung der allgemeinen Formel (RO)_{q}AlR'ᵣ setzt, bei der R ein Hydrocarbylrest ist, welcher 1 bis 40 Kohlenstoffatome enthält und R' ein Alkylrest ist, der 1 bis 20 Kohlenstoffatome enthält, wobei q und r gleich 1 oder 2 derart sind, dass q + r gleich 3 ist und dass das erhaltene Produkt getrocknet wird.

7. Verfahren zur Herstellung eines modifizierten Katalysators nach Anspruch 6, bei dem die Verbindung (RO)_{q}AlR'ᵣ aus der Reaktion zwischen einer Verbindung ROH und einer Verbindung AlR'₃ resultiert.

8. Verfahren zur Herstellung eines modifizierten Katalysators, einem mineralischen porösen Träger, 0,01 bis 20 Gew.-% Rhenium in Form des Oxids und 0,01 bis 10 Gew.-% Aluminium umfassend, bei dem man einen kalzinierten Vorläufer herstellt, der den mineralischen porösen Träger und das Rhenium enthält, dann diesen Vorläufer mit Vorläuferreaktionsmitteln der Verbindung der allgemeinen Formel (RO)_{q}AlR'ᵣ kontaktiert, in der R ein Hydrocarbylrest, der 10 bis 40 Kohlenstoffatome enthält, und R' ein Alkylrest ist, der 1 bis 20 Kohlenstoffatome enthält und wobei q und r gleich 1 oder 2 derart sind, dass q + r gleich 3 ist und dass das erhaltene Produkt getrocknet wird.

9. Verfahren zur Herstellung eines modifizierten Katalysators nach Anspruch 8, bei dem die Vorläuferreaktionsmittel der Verbindung (RO)_{q}AlR'ᵣ die Verbindungen ROH und AlR'₃ sind.

10. Verfahren nach einem der Ansprüche 6 bis 9, bei dem der mineralische poröse Träger aus der durch Aluminiumoxid, Siliziumoxid, die Siliziumoxide-Aluminiumoxide, die Zeolithe, das Titanoxid, das Zirkonoxid, das Nioboxid, das Oxid des Chroms, das Magnesiumoxid, das Zinnoxid gebildeten Gruppe gewählt ist.

11. Verfahren nach einem der Ansprüche 6 bis 10, bei dem der mineralische poröse Träger gewählt ist aus der durch Aluminiumoxid und die Verbindungen, die wenigstens 75 Gew.-% Aluminiumoxid enthalten, gebildeten Gruppe und über eine Oberfläche von wenigstens 10 m²/g und ein Porenvolumen von wenigstens 0,1 ml/g verfügt.

12. Verfahren nach einem der Ansprüche 6 bis 11, bei dem der Katalysatorvorläufer erhalten wird durch Kontakt des Trägers mit wenigstens einer Rheniumverbindung, die als Vorläufer verwendet wird, gewähit aus der durch Rheniumheptoxid, Ammoniumperrhenat und die perrhenische Säure gebildeten Gruppe.

13. Verfahren nach Anspruch 12, **dadurch gekennzeichnet, dass** nach Zugabe des Rheniums auf den Träger eine Kalzinierung bei einer Temperatur von 200 bis 1000°C über eine Zeitdauer von 10 Minuten bis 10 Stunden vorgenommen wird.

14. Verfahren nach einem der Ansprüche 6 bis 13, **dadurch gekennzeichnet, dass** die Aluminiumverbindung der allgemeinen Formel (RO)_{q}AlR'ᵣ entspricht, in der R ein Hydrocarbylrest ist, der gewählt ist aus der durch die Alkyl-, Zykloalkyl-, Alkenyl-, Aryl-, Arlyl- oder Zykloalkylreste, die letzten beiden in Substitution, gebildeten Gruppe gewählt ist, wobei dieser Rest durch wenigstens eine Alkoxygruppe oder wenigstens ein Halogen substituiert werden kann.

15. Verfahren nach einem der Ansprüche 6 bis 14, **dadurch gekennzeichnet, dass** das erhaltene Produkt nach Kontakt mit der Verbindung der Formel (RO)_{q}AlR'ᵣ, oder mit ihren Vorläufern getrocknet wird bei einer Temperatur von weniger als 200°C.

16. Verfahren zur Metathese der Olefine, **dadurch gekennzeichnet, dass** man bei einer Temperatur von -20 bis +200°C und in Anwesenheit eines Katalysators nach einem der Ansprüche 1 bis 5 arbeitet.

17. Verfahren zur Metathese der Olefine, **dadurch gekennzeichnet, dass** man bei einer Temperatur von -20 bis +200°C und in Anwesenheit eines nach einem der Ansprüche 6 bis 14 erhaltenen Katalysators arbeitet.

18. Verfahren nach einem der Ansprüche 16 bis 17, **dadurch gekennzeichnet, dass** man zwischen 0 und 100°C arbeitet.

19. Verfahren nach einem der Ansprüche 16 bis 18, **dadurch gekennzeichnet, dass** die Olefine gewählt sind aus der Gruppe, die durch die Monoolefine mit 2 bis 30 Kohlenstoffatomen, die Zykloolefine mit 3 bis 20 Kohlenstoffatomen, die Polyolefine mit 4 bis 30 Kohlenstoffatomen, die Zyklopolyolefine mit 5 bis 30 Kohlenstoffatomen, die Monoolefine mit 2 bis 30 Kohlenstoffatomen, welche funktionale Gruppen tragen, die in der durch die Halogene gebildeten Gruppen und die Estergruppen gewählt sind, die Polyolefine mit 4 bis 30 Kohlenstoffatomen, welche funktionale Gruppen tragen, die in der durch die Halogene und die Estergruppen gebildeten Gruppe gewählt sind, gebildet ist.

20. Verfahren nach einem der Ansprüche 16 bis 19, **dadurch gekennzeichnet, dass** die Olefine gewählt sind aus der durch Polyethylen, Polypropylen, die Butene, die Pentene, Zyklopenten, Zyklookten, Norbornen, 1,3-Hexadien, 1,7-Oktadien, 1,5 Zyklooktadien, Norbornadien, Dizyklopentadien, Methyloleat gebildeten Gruppe.

21. Verfahren nach einem der Ansprüche 16 bis 20, **dadurch gekennzeichnet, dass** es in gasförmiger Phase abläuft.

22. Verfahren nach einem der Ansprüche 16 bis 20, **dadurch gekennzeichnet, dass** die Reaktionsteilnehmer und das gegebenenfalls vorhandene Lösungsmittel wenigstens zum Teil in flüssiger Phase vorliegen.
